(19) **Europäisches Patentamt / European Patent Office / Office européen des brevets**

(11) **EP 4 317 156 A1**

(12) **EUROPEAN PATENT APPLICATION**
published in accordance with Art. 153(4) EPC

(43) Date of publication:
07.02.2024 Bulletin 2024/06

(21) Application number: 22778630.8

(22) Date of filing: 21.03.2022

(51) International Patent Classification (IPC):
$C07D\ 417/12^{(2006.01)}$   $A61K\ 9/10^{(2006.01)}$
$A61K\ 47/10^{(2017.01)}$   $A61K\ 31/496^{(2006.01)}$
$A61P\ 25/00^{(2006.01)}$   $A61P\ 25/18^{(2006.01)}$
$A61P\ 25/24^{(2006.01)}$

(52) Cooperative Patent Classification (CPC):
A61K 9/10; A61K 31/496; A61K 47/10;
A61P 25/00; A61P 25/18; A61P 25/24;
C07D 417/12

(86) International application number:
PCT/CN2022/081937

(87) International publication number:
WO 2022/206447 (06.10.2022 Gazette 2022/40)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 31.03.2021 CN 202110348916

(71) Applicant: Sichuan Kelun Pharmaceutical
Research
Institute Co., Ltd.
Chengdu, Sichuan 611138 (CN)

(72) Inventors:
• LI, Ming
  Chengdu, Sichuan 611138 (CN)
• LIANG, Xiangyong
  Chengdu, Sichuan 611138 (CN)

• SU, Zhengxing
  Chengdu, Sichuan 611138 (CN)
• LI, Dan
  Chengdu, Sichuan 611138 (CN)
• KE, Duo
  Chengdu, Sichuan 611138 (CN)
• YI, Cong
  Chengdu, Sichuan 611138 (CN)
• WEI, Wei
  Chengdu, Sichuan 611138 (CN)
• DENG, Guifu
  Chengdu, Sichuan 611138 (CN)
• PENG, Ya
  Chengdu, Sichuan 611138 (CN)
• ZHAO, Dong
  Chengdu, Sichuan 611138 (CN)
• WANG, Jingyi
  Chengdu, Sichuan 611138 (CN)

(74) Representative: Cabinet Nony
11 rue Saint-Georges
75009 Paris (FR)

(54) **INJECTABLE LURASIDONE SUSPENSION AND PREPARATION METHOD THEREFOR**

(57) The present disclosure relates to an injectable lurasidone suspension and a preparation method thereof, and in particular to an irregular form of a lurasidone solid and a pharmaceutical composition thereof. The present disclosure also relates to a preparation method for the solid and the pharmaceutical composition thereof, and an application thereof in the treatment of mental diseases. According to the present disclosure, the lurasidone solid prepared has controllable particle size and has Dv50 particle size of $6\mu m$ to $110\mu m$. The good particle size stability can also be maintained in the pharmaceutical composition. The lurasidone suspension preparation obtained by the method is fast-acting, has a long sustained release period, and can effectively reduce the risk caused by poor patient compliance.

EP 4 317 156 A1

**Fig. 2**

**Description**

The present application is based on the application with CN application number of 202110348916.6 and the application date of March 31, 2021, and claims its priority. The content of this CN application is hereby incorporated into the present application in its entirety.

**Technical Field**

The present invention relates to the field of pharmaceutical preparation, in particular to an injectable lurasidone suspension and a method for preparing the same.

**Background Art**

Lurasidone is a dopamine D2 and 5-HT2A receptor antagonist, a 5-HT7 receptor partial antagonist, and a 5-HT1A receptor partial agonist, which is developed by Japan Sumitomo Pharmaceutical Co., Ltd. (hereinafter referred to as Sumitomo Japan), and is an atypical antipsychotics. It was first released in tablet form in the United States in 2011 for the treatment of schizophrenia. Compared with other marketed atypical antipsychotic drugs, lurasidone has the safety advantage of reducing the risk of extrapyramidal adverse reactions and QT interval prolongation, and is a first-line drug for the treatment of schizophrenia. However, lurasidone tablets still face many problems in the clinical use. On the one hand, due to the protracted course of schizophrenia, it is necessary to take drugs for a long time; on the other hand, part of the cognitive function of the patients is impaired, and the compliance is poor, which often leads to the phenomenon of missing or even discontinuation of medication; in addition, lurasidone has a low oral bioavailability (9%-19%) and a high frequency of administration (once a day), which further increases the risk of missing or even discontinuation of medication. Therefore, the development of lurasidone long-acting injection (> 7 days) has important clinical significance.

In the existing long-acting preparation technologies, injectable suspension has unique advantages in terms of process simplicity and drug loading capacity, and has gradually become the preferred dosage form in the field of chronic diseases, especially in the field of mental diseases. Injectable suspensions are usually prepared by "top-down" (e.g., medium grinding method), "bottom-up" (e.g., solvent precipitation), or a combination of both (e.g., raw material with large particle size is first obtained by solvent precipitation method, and then treated by medium grinding method to a desired particle size). China Pharmaceutical University has successfully prepared lurasidone hydrochloride nanosuspension (CN 109998991A) by a wet medium grinding method (planetary ball mill), but its sustained-release period is only 3-7 days, and its clinical advantages are not significantly. By using the same technology, LifeMax Laboratories, Inc. has developed a long-acting lurasidone preparation (WO 2019/213286 A1), with excellent syringeability, which can achieve a sustained-release effect of at least 28 days (particle size range: 4 ~100$\mu$m). However, there is a risk of introducing medium fragments during the preparation process of wet medium grinding method, and it is necessary to connect the grinder system to the liquid distribution system for cleaning and sterilization during scale-up production, which increases the complexity and production cost of the production system. It may be for this reason that Sumitomo Japan adopted the "bottom-up" preparation method when developing lurasidone sustained-release preparations (CN 103249416B). It is prepared by adding the organic phase solution containing lurasidone dropwise into the aqueous solution containing surfactants (i.e., back-dropping method) to prepare a lurasidone sustained-release preparation with a particle size of 4-26$\mu$m (the particle is of cubic shape), which can achieve a sustained-release effect of at least more than 2 to 4 weeks. In this preparation process, it is necessary to control all parameter systems related to the crystallization process to ensure the controllability of the particle size distribution of the final product (particle size distribution is the primary quality indicator of injectable suspensions and must be controllable), including stirring speed, crystallization temperature, organic phase/aqueous solution volume ratio, dropping rate, etc., even an additional bypass cycle is required for better control of product particle size. The complex parameter systems bring great challenges to control the particle size distribution of product (i.e., the controllability of product quality). In addition, the preparation published by CN 103249416B releases slowly in the early stage of the body (e.g., 4 days after the intramuscular injection, the preparation No.4 in Example 12 showed a blood concentration of about 50% of $C_{max}$). In clinical practice, it is very likely that similar tablets need to be taken orally a few days before the intramuscular injection to make the blood concentration reach the therapeutic level quickly, and there is still the risk of missing medicament in patients.

**Contents of the present invention**

The present invention provides a method for preparing lurasidone, which only needs to control the temperature and dispersion conditions (stirring or shearing) during recrystallization of lurasidone to achieve the goal of controlling the particle size of lurasidone. Moreover, we unexpectedly found that the crystal form of lurasidone prepared by the present invention presents an irregular form, which leads to a rapid increase of blood drug concentration after intramuscular injection (the blood drug concentration reaches 62% of $C_{max}$ after about 4 hours), then sustained-releases is followed, the effective period of sustained-release can be as long as 30 days, and thus it works quickly in clinical use without additional oral tablets, which greatly solves the problem of patient compliance.

In one aspect, the present invention provides a lurasidone solid, which has a powder X-ray diffraction pattern showing the following diffraction angles 2θ(°): 15.1±0.2, 15.5±0.2, 16.3±0.2, 16.6±0.2, 18.0±0.2 0.2 and 20.0±0.2, and the solid is in irregular form.

The "irregular form" mentioned herein means that it does not have a standard three-dimensional shape, and there is no obvious similarity between the shapes of the particles. The standard three-dimensional shape refers to a standard three-dimensional shape such as cube, cuboid, cylinder, and cone commonly seen. In some embodiments, the solid has a random three-dimensional shape observed with a polarizing microscope (e.g., CiPOL, Nikon). In some embodiments, the three-dimensional shapes of the solids of the present invention are not completely identical observed with a polarizing microscope (e.g., CiPOL, Nikon).

In some embodiments, the solid has a powder X-ray diffraction pattern showing the following diffraction angles 2θ(°): 11.2±0.2, 15.1±0.2, 15.5±0.2, 16.3±0.2, 16.6±0.2, 18.0±0.2, 19.1 ±0.2, 19.5±0.2, 20.0±0.2, 20.9±0.2, 22.3±0.2 and 26.0±0.2.

In some embodiments, the solid has a Dv50 particle size of 6μm to 110μm.

In some embodiments, the solid has a Dv50 particle size of 6μm to 100μm, 6μm to 90μm, 6μm to 80μm, 6μm to 70μm, 6μm to 60μm, 6μm to 50μm, 6μm to 40μm, 6μm to 30μm, 6μm to 20μm, 6μm to 10μm, 10μm to 100μm, 10μm to 90μm, 10μm to 80μm 10μm to 70μm, 10μm to 60μm, 10μm to 50μm, 10μm to 40μm, 10μm to 30μm, 10μm to 20μm, 20μm to 100μm, 20μm to 90μm, 20μm to 80μm 20μm to 70μm, 20μm to 60μm, 20μm to 50μm, 20μm to 40μm, 20μm to 30μm, 20μm to 100μm, 20μm to 90μm, 20μm to 80μm 20μm to 70μm, 20μm to 60μm, 20μm to 50μm, 20μm to 40μm or 20μm to 30μm.

In some embodiments, the solid has a melting point of 148±5°C, such as 148±4°C, 148±3°C, 148±2°C, 148±1.5°C, 148±1°C, 148±0.9°C, 148±0.8°C, 148±0.7 148±0.6°C, 148±0.5°C, 148±0.4°C, 148±0.3°C, 148±0.2°C or 148±0.1°C.

In another aspect, the present invention provides a method for preparing the lurasidone solid, which comprises the following steps:

(1) dissolving lurasidone in an organic solvent under heating to obtain a solution of lurasidone;

(2) cooling the solution obtained in step (1) to room temperature (e.g., 25 °C) to precipitate crystals;

(3) collecting the crystals obtained in step (2) to obtain the lurasidone solid;

optionally, during the cooling process as described in step (2), controlling stirring speed to 100 rpm to 2000 rpm, and/or shear line speed to 1 m/s to 32 m/s.

In some embodiments, the heating in step (1) refers to heating to a degree sufficient to increase the solubility of lurasidone in the organic solvent. Herein, unless otherwise specified, the solubility refers to the mass of lurasidone dissolved in 100 g of solvent when it reaches saturation at a specific temperature (e.g., 25 °C). In some embodiments, the heating in step (1) refers to heating to the boiling point of the organic solvent or to the temperature within ±1°C to ±30°C of its boiling point, for example within ±1°C, ±2°C, ±3°C, ±4°C, ±5°C, ±6°C, ±7°C, ±8°C, ±9°C, ±10°C, ±15°C, ±20°C, ±25°C or ±30°C of its boiling point.

In some embodiments, the organic solvent as described in step (1) is the Class III solvent classified according to ICH. In some preferred embodiments, the organic solvent is selected from the group consisting of ethanol, n-propanol, isopropanol, isopropyl acetate, methyl acetate, ethyl acetate, acetic acid, 1-pentanol, 1-butanol and 2-butanol. In some preferred embodiments, the organic solvent is selected from the group consisting of ethanol, n-propanol, isopropanol and any combination thereof. In some preferred embodiments, the organic solvent is ethanol or isopropanol.

In some embodiments, after step (1) and before step (2), a step of filtering and sterilizing the solution, for example, a step of filtering and sterilizing by using a 0.22μm filter membrane, is further comprised.

By controlling the cooling process, the purpose of controlling the particle size of the product can be achieved.

Constant velocity cooling is applied by keeping the cooling rate (0.05°C/min to 15°C/min) unchanged; alternatively, more than two cooling rates can be comprised in the entire cooling process.

In some embodiments, before and after reaching the crystallization temperature (or near the crystallization temperature, for example, within ±5°C, ±4°C, ±3°C, ±2°C, ±1°C, or ±0.5°C of the crystallization temperature), a first cooling rate and a second cooling rate are set, respectively. In some embodiments, the first cooling rate is 0.05°C/min to 0.1°C/min, and the second cooling rate is 0.1°C/min to 5°C/min (e.g., 0.2 to 0.5 °C/min). In some embodiments, the cooling rate is set to 0.08°C/min at 80°C to 60°C, and to 3°C/min at 60°C to 25°C. Alternatively, by keeping a cooling medium at a constant temperature, the cooling rate gradually decreases from high to low, the greater the temperature difference between the organic solution and the medium, the faster the cooling rate, and during the process that the temperature of the organic solution gradually approaches the temperature of the medium, the cooling rate gradually decreases; theoretically, the temperature of the cooling medium is set in a range from the melting point of the organic solvent used to the boiling point of the organic solvent used; however, by considering the operability, yield, etc., the actual temperature of the cooling medium can be set to -20°C to 70°C. During the entire cooling process, one or more temperatures of the cooling medium can be set; for example, the cooling medium temperature is firstly set as 60°C, and after the organic solution reaches 60°C, the cooling medium temperature is kept at 25°C until the organic solution temperature drops to 25°C.

Optionally, the cooling process in step (2) can be realized by a combination of constant cooling rate and constant cooling medium temperature.

Theoretically, the lowest temperature can be reduced to around the melting point of the organic solvent used (e.g., the melting point of ethanol is -114°C, when ethanol is used as the organic solvent, the theoretical minimum temperature can be reduced to around -110°C), but in practice, it is found that when the organic solution is cooled to room temperature, the yield of about 90% could be achieved, that is, excessive cooling has no obvious benefit, so it is preferable to lower the temperature to room temperature (25°C) to reduce production energy consumption.

In some embodiments, cooling the solution obtained in step (1) to room temperature at the following rate: 0.05°C/min to 15°C/min, 0.05°C/min to 10°C/min, 0.05°C/min to 5°C/min, 0.05°C/min to 3°C/min, 0.05°C/min to 2°C/min, 0.05°C/min to 1°C/min, 0.05°C/min to 0.5°C/min, 0.05°C/min to 0.2°C/min, or 0.05°C/min to 0.1°C/min.

In other embodiments, the solution obtained in step (1) is cooled to room temperature by setting the temperature of the cooling medium: the temperature of the cooling medium is set in a range of -20 to 70°C, and one or more cooling medium temperatures can be set during the entire cooling process according to the actual situation; thereby dividing one or more cooling stages. In some embodiments, two cooling stages are set in the entire cooling process, and the temperature of the cooling medium in the first cooling stage is 50% to 90% of the heating temperature in step (1) (e.g., 50% to 60%, 50% to 70%, 50% to 80%, 60% to 70%, 60% to 80%, 60% to 90%, 70% to 80%, 70% to 90%, or 80% to 90%); the temperature of the cooling medium in the second cooling stage is at room temperature (e.g., 25°C). For example, the temperature of the cooling medium is directly set as 25°C, so that the temperature of the solution obtained in step (1) gradually drops to room temperature (25°C, the entire cooling process is a cooling stage); for another example, the temperature of the cooling medium is set as 60°C, and after the solution obtained in step (1) is cooled to about 60°C (this is the first cooling stage), the temperature of the cooling medium is set as 25°C again, so that the solution obtained in step (1) is continuously cooled to room temperature (25°C, this is the second cooling stage).

During the cooling process, the particle size of the product can be further controlled by adjusting the stirring speed or the shear line speed. In some embodiments, during the cooling process as described in step (2), the stirring speed is controlled to 100rpm to 1000rpm, such as 200rpm to 1000rpm, 300rpm to 1000rpm, 400rpm to 1000rpm, 500rpm to 1000rpm, 600rpm to 1000rpm, 700rpm to 1000rpm, 800rpm to 1000rpm, or 900rpm to 1000rpm.

In some embodiments, during the cooling process as described in step (2), the shear line speed is controlled to 1m/s to 16m/s, such as 1.5m/s to 16m/s, 2m/s to 16m/s, 2.5m/s to 16m/s, 3m/s to 16m/s, 3.5m/s to 16m/s, 4m/s to 16m/s, 4.5m/s to 16m/s, 5m/s to 16m/s, 5.5m/s to 16m/s, 6m/s to 16m/s, 6.5m/s to 16m/s, 7m/s to 16m/s, 7.5m/s to 16m/s, 8m/s to 16m/s, 8.5m/s to 16m/s, 9m/s to 16m/s, 9.5m/s to 16m/s, 10mls to 16m/s, 10.5m/s to 16m/s, 11m/s to 16m/s, 11.5m/s to 16m/s, 12m/s to 16m/s, 12.5m/s to 16m/s, 13m/s to 16m/s, 13.5m/s to 16m/s, 14m/s to 16m/s, 14.5m/s to 16m/s, 15m/s to 16m/s, or 15.5m/s to 16m/s.

In some other optimized embodiments, when the temperature of the solution obtained in step (1) drops to a specific temperature, a shearer is turned on for crystallization. The specific temperature refers to 0 to 5°C higher than the crystallization temperature, for example, 0 to 0.5°C, 0 to 1°C, 0 to 1.5°C, 0 to 2°C, 0 to 2.5°C, 0 to 3°C, 0 to 3.5°C, 0 to 4°C, 0 to 4.5°C, 0 to 5°C, 0.5 to 1°C, 0.5 to 1.5°C, 0.5 to 2°C, 0.5 to 2.5°C, 0.5 to 3°C, 0.5 to 3.5°C, 0.5 to 4°C, 0.5 to 4.5°C, 0.5 to 5°C, 1 to 1.5°C, 1 to 2°C, 1 to 2.5°C, 1 to 3°C, 1 to 3.5°C, 1 to 4°C, 1 to 4.5°C, 1 to 5°C, 1.5 to 2°C, 1.5 to 2.5°C, 1.5 to 3°C, 1.5 to 3.5°C, 1.5 to 4°C, 1.5 to 4.5°C, 1.5 to 5°C, 2 to 2.5°C, 2 to 3°C, 2 to 3.5°C, 2 to 4°C, 2 to 4.5°C, 2 to 5°C, 2.5 to 3°C, 2.5 to 3.5°C, 2.5 to 4°C, 2.5 to 4.5°C, 2.5 to 5°C, 3 to 3.5°C, 3 to 4°C, 3 to 4.5°C, 3 to 5°C, 3.5 to 4°C, 3.5 to 4.5°C, 3.5 to 5°C, 4 to 4.5°C, 4 to 5°C, 4.5 to 5°C higher than the crystallization temperature.

By controlling the aforementioned cooling rate and/or further controlling the stirring speed or the shear line speed during the cooling process, the obtained lurasidone solid has a controllable particle size in the range of 6μm to 110μm (the particle size refers to a particles size corresponding to 50% volume distribution, i.e., Dv50, which is measured by Malvern Mastersizer3000 wet method), preferably 10μm to 30μm.

When the organic solvent used is soluble in water, after the precipitated crystals are collected by filtration, the crystals can be washed directly with a large amount of water for injection to remove the residual organic solvent. During the washing process, the filter cake can be scraped up and mixed with the washing agent thoroughly to improve the washing efficiency, in order to promote the mixing of the filter cake and the washing agent, the washing agent may contain 0.01% to 5% (W/W) HS 15 or similar surfactants; the amount of the washing agent is 0.5 to 10 times that of volume of the organic solution. After washing, the obtained filter cake can be directly dispersed in a solution of the pharmaceutical excipient as described herein, the solid concentration of lurasidone is 10% to 50% (W/W), preferably 20% to 40% (W/W ), and it can be packed according to different specifications for clinical needs to obtain the final product available for clinical use.

In some embodiments, in step (3), after collecting the crystals obtained in step (2), a drying step is further comprised, for example, heating drying or heating vacuum drying, preferably vacuum drying at 40 to 70 °C for 6 to 24h; more preferably vacuum drying at 40 to 60 °C for 6 to 12h. In order to prevent the material from agglomerating, stirring the material may be operated during the drying process.

The dried sterile raw materials are subpackaged according to unit doses, and the unit dose ranges from 50 mg to 500 mg, and a certain amount of pharmaceutical excipient solution can be added before use. The dried sterile raw material can also be made into a preparation product that can be used directly with the pharmaceutical excipient solution, and the solid concentration of lurasidone is 10% to 50% (W/W), preferably 20% to 40% (W/W), and it can be packed according to different specifications for clinical needs to obtain the final product available for clinical use.

If the parameter control in the preparation process directly affects the controllability of the quality, the composition of the formulation of the preparation (e.g., injectable suspension) directly affects the stability of the quality. The present invention provides a pharmaceutical composition with excellent stability, which comprises the aforementioned lurasidone solid, and a pharmaceutical excipient, such as one or more of a surfactant, a pH buffering agent, a dispersing agent, a pH regulator, an osmotic pressure regulator and a stabilizer.

In some embodiments, the content of the lurasidone solid accounts for 10% to 50%, for example, 10%, 15%, 20%, 25%, 30%, 35%, 40%, 45% or 50%, preferably 20% to 40% (W/W) of the pharmaceutical composition.

The surfactant has a wetting agent effect, and in the present invention, a nonionic surfactant is particularly preferred, including Kolliphor® HS 15, polysorbate 80, polysorbate 20, polyoxyethylene castor oil, sorbitan fatty acid ester, succinate, etc., in which, Kolliphor® HS 15 is originally developed by BASF in Germany (the original trade name is Solutol® HS 15, hereinafter referred to as HS 15). The structure of HS 15 type nonionic surfactants is characterized in that one or both ends of polyethylene glycol are linked by fatty acid chains via ester bonds, the number of carbon atoms in the fatty acid chain is 8 to 26 (having 0 to 3 unsaturated bonds), and there is at least one hydroxy group on the fatty acid chain, or some free polyethylene glycols are contained at the same time. At present, HS 15 is mainly used as a solubilizer in pharmaceutical preparations, and it has not been reported to be used as a wetting agent in injectable suspension preparations. We unexpectedly found that compared with commonly used wetting agents, nonionic surfactants represented by HS 15 showed a better wetting effect on lurasidone, and better particle size stability under the same conditions (combined with the same stabilizer).

In some embodiments, the surfactant is a nonionic surfactant such as one or more of polysorbate 80, polysorbate 20, polyoxyethylene castor oil, sorbitan fatty acid ester, succinate, and HS 15, preferably HS 15. In some embodiments, the content of the surfactant accounts for 0.01% to 5%, such as 0.01%, 0.02%, 0.04%, 0.06%, 0.08%, 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5% or 5%, preferably 0.5% to 2% (W/W) of the pharmaceutical composition.

In some embodiments, the pH buffering agent is one or more selected from the group consisting of sodium phosphate, disodium hydrogen phosphate, sodium dihydrogen phosphate, potassium phosphate, dipotassium hydrogen phosphate, potassium dihydrogen phosphate, tromethamine, sodium carbonate, sodium acetate, sodium bicarbonate, meglumine, arginine, triethanolamine, citric acid and acetic acid; preferably a combination of sodium dihydrogen phosphate and disodium hydrogen phosphate. In some embodiments, the content of the pH buffering agent accounts for 0.01% to 5%, such as 0.01%, 0.02%, 0.04%, 0.06%, 0.08%, 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5% or 5% (W/W) of the pharmaceutical composition.

In some embodiments, the dispersing agent is an aqueous medium, preferably water for injection.

In some embodiments, the pH regulator is selected from the group consisting of sodium hydroxide, potassium hydroxide, hydrochloric acid, and aqueous solution thereof. The pH regulator has a content that makes pH of the pharmaceutical composition 4 to 9, such as 4, 5, 6, 7, 8 or 9, preferably 7.

In some embodiments, the osmotic pressure regulator is selected from the group consisting of sodium chloride, glucose, and mannitol. In some embodiments, the content of the osmotic pressure regulator accounts for 0.1% to 10%, such as 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 5.5%, 6%, 6.5%, 7%, 7.5 %, 8%, 8.5%, 9%, 9.5% or 10% (W/W) of the pharmaceutical composition.

In some embodiments, the stabilizer is selected from the group consisting of polyethylene glycol stabilizer (e.g., polyethylene glycol 4000), carboxymethylcellulose and sodium salt thereof, polyvinylpyrrolidone (e.g., PVP K17, PVP K30), hypromellose, methylcellulose, poloxamer (e.g., 188, 407), and polyvinyl alcohol. In some embodiments, the content of the stabilizer accounts for 0.01% to 25%, such as 0.01%, 0.02%, 0.04%, 0.06%, 0.08%, 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5%, 10%, 15%, 20% or 25% (W/W) of the pharmaceutical composition.

In some embodiments, the pharmaceutical composition can pass through a syringe needle with an inner diameter of 0.3 mm to 1 mm (e.g., about 0.3 mm, about 0.4 mm, about 0.5 mm, about 0.6 mm, about 0.7 mm, about 0.8 mm, about 0.9 mm, about 1mm), such as a 18G to 23G syringe needle according to international general standard specifications.

In some embodiments, the pharmaceutical composition is an injection, preferably a long-acting sustained-release injection, such as a suspension injection or a lyophilized preparation.

In some embodiments, the pharmaceutical composition exists in the form of a unit preparation, and further preferably, the unit preparation contains 50 mg to 500 mg of the lurasidone solid.

In some embodiments, the pharmaceutical composition has the weight composition of:

| | |
|---|---|
| Lurasidone solids | 30%; |
| Kolliphor® HS 15 | 1.2%; |
| Sodium dihydrogen phosphate | 0.24%; |
| Disodium hydrogen phosphate | 0.48%; |
| Sodium carboxymethyl cellulose | 1.25%; and |
| Water for injection | balanced. |

In another aspect, the present invention provides a method for preparing the aforementioned pharmaceutical composition, which comprises the following steps:
dispersing the lurasidone solid into the pharmaceutical excipient to obtain the pharmaceutical composition.

In some embodiments, during the dispersing process, an operation of stirring, shearing, sonication, or any combination thereof is conducted.

In some embodiments, the speed of the stirring is 100 rpm to 2000 rpm.

In some embodiments, the line speed of the shearing is 1 m/s to 32 m/s.

In some embodiments, the frequency of the sonication is 20 to 60KHz.

In another aspect, the present invention provides use of the lurasidone solid or the pharmaceutical composition described herein in the manufacture of a medicament for the treatment of a mental disease. In some embodiments, the mental disease is selected from the group consisting of schizophrenia, bipolar disorder, and depression.

In another aspect, the present invention provides the lurasidone solid or the pharmaceutical composition as described herein, for use in the treatment of a mental disease. In some embodiments, the mental disease is selected from the group consisting of schizophrenia, bipolar disorder, and depression.

In another aspect, the present invention provides a method of treating a mental disease, which comprises administering to a subject in need thereof a therapeutically effective amount of the lurasidone solid or the pharmaceutical composition described herein. In some embodiments, the mental disease is selected from the group consisting of schizophrenia, bipolar disorder, and depression.

Explanation of the terms

Unless defined otherwise, all technical and scientific terms used herein have the same meaning as commonly understood by those skilled in the art. References to techniques used herein are intended to refer to techniques commonly understood in the art, including those variations of techniques that are obvious to those skilled in the art or substitutions of equivalent techniques. Although the following terms are believed to be well understood by those skilled in the art, the following definitions are set forth to better explain the present invention.

As used herein, the terms "including", "comprising", "having", "containing" or "involving" and other variations thereof herein are inclusive or open-ended, and other unlisted elements or method steps are not excluded.

As used herein, the term "crystallization temperature" refers to a temperature at which crystals begin to precipitate out of the lurasidone solution obtained in step (1) under certain cooling conditions.

The singular forms "a/an" and "the" include plural referents unless the content clearly dictates otherwise.

As used herein, the term "individual" or "subject" is a mammal, such as a bovine, equine, porcine, canine, feline, rodent, primate; wherein, the particularly preferred subject is a human.

As used herein, the term "effective amount" refers to an amount sufficient to achieve, or at least partially achieve, the desired effect. For example, a therapeutically effective amount is an amount sufficient to cure or at least partially prevent a disease and complication thereof in a patient already suffering from the disease. Determining such an effective amount is well within the capability of those skilled in the art. For example, a therapeutically effective amount will depend on the severity of the disease to be treated, the general state of the patient's own immune system, the patient's general conditions such as age, body weight and gender, the mode of administration of the drug, and other therapy administered concomitantly etc.

The amount of drug administered to a subject depends on the type and severity of the disease or condition and the characteristics of the subject, such as general health, age, gender, body weight and tolerance to the drug, and also on factors such as the formulation type and route of administration of the drug, as well as the dosing cycle or time interval between administrations. Based on these and other factors, one skilled in the art will be able to determine an appropriate dosage. The preparation of the present invention can be administered parenterally with a solid dose of lurasidone ranging from 50 mg to 1200 mg (preferably 100 mg to 800 mg), particularly preferably administered intramuscularly or subcutaneously as an injectable suspension. The administration frequency of the pharmaceutical composition of the present invention may be once every two to four weeks, preferably once every four to six weeks, more preferably once every four weeks.

Beneficial effects of the present invention

The present invention relates to a lurasidone solid in irregular form and a pharmaceutical composition thereof, a method for preparing the solid and pharmaceutical composition thereof, and application thereof in the treatment of a mental disease. In the process for preparing the lurasidone solid, a single-solvent system is used, and the particle size of lurasidone can be controlled by simple parameter control, and its particle morphology is obviously different from the disclosed cubic form, showing an irregular form, and finally a final product with better safety and stability is obtained; after intramuscular injection, the blood drug concentration rises rapidly, and then releases slowly, which can achieve a sustained-release effect of at least 30 days. In clinical use, it works quickly without oral tablets, which can effectively reduce the risk of poor patient compliance and solve the problem of patient compliance to the greatest extent. In addition, the surfactant represented by Kolliphor® HS 15 (HS 15 for short) used in the present invention has more advantages in terms of safety and stability, and also shows better wetting effect and particle size stability on lurasidone formulations.

**Brief Description of the Drawings**

The accompanying drawings described herein are used to provide a further understanding of the present invention and constitute a part of the present application. The schematic examples of the present invention and their descriptions are used to explain the present invention and do not constitute an inappropriate limitation on the present invention. In the drawings:

Fig. 1 shows the XRD spectrum of the crystals prepared in Example 1.

Fig. 2 shows the polarizing microscope photo (magnified 200 times) of the crystals prepared in Example 1.

Fig. 3 shows the DSC results (35°C to 250°C, 10°C/min) of the crystals prepared in Example 1.

Fig. 4 shows the XRD spectrum of the crystals prepared in Example 3.

Fig. 5 shows the polarizing microscope photo (magnified 200 times) of the crystals prepared in Example 3.

Fig. 6 shows the DSC spectrum (35°C to 250°C, 10°C/min) of the crystals prepared in Example 3.

Fig. 7 shows the in vivo pharmacokinetic results of intramuscular injection of the lurasidone suspension (50 mg/Kg) of Example 10 in SD rats.

Fig. 8 shows the in vivo pharmacokinetic results of intramuscular injection of the lurasidone suspension (50 mg/Kg) of Comparative Example 1 in SD rats.

Fig. 9 shows the observation results of stability of particle size of Sample 12.

**Specific Models for Carrying Out the present invention**

The following will clearly and completely describe the technical solutions in the examples of the present invention with reference to the accompanying drawings in the examples of the present invention. Obviously, the described examples are only some, not all, examples of the present invention. The following description of at least one exemplary example is merely illustrative in nature and in no way taken as any limitation of the present invention, its applications or uses. Based on the examples of the present invention, all other examples obtained by those skilled in the art without creative efforts fall within the protection scope of the present invention.
For examples without indicating the specific conditions, they were carried out according to the conventional conditions or the conditions suggested by the manufacturer. For reagents or instruments used without indicating manufacturer, they were all commercially available conventional products.

Unless otherwise specified, the lurasidone described herein refers to lurasidone free base.

Example 1

200g of lurasidone was weighed, added into 4L of absolute ethanol, and stirred at 80°C until completely dissolved, the solution was cooled to 60°C by controlling the cooling rate at about 0.05°C/min, then the solution was cooled to room temperature (25°C) by controlling the cooling rate at about 0.02°C/min, the precipitated crystals were filtered, subjected to suction filtration, followed by vacuum drying at 60°C for 12 hours (-0.07MPa to -0.08MPa), and the yield was 96%.

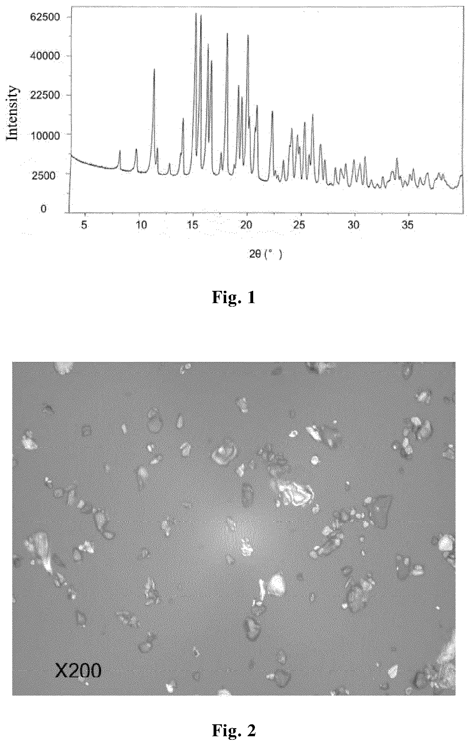

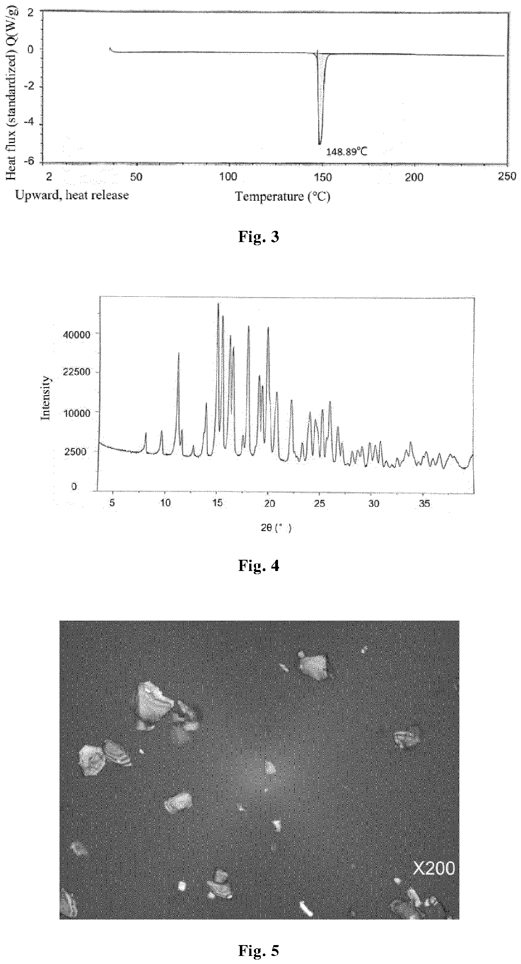

The obtained lurasidone had XRD results as shown in Figure 1, and it was found that the lurasidone had irregular form observed with a polarizing microscope (CiPOL, Nikon) (Figure 2), it was obviously different from the cubic crystals disclosed in CN103249416A; the DSC results showed that the melting point was 148.89°C (Figure 3); the particle size was 16.320$\mu$m (Dv50, measured by Malvern Mastersizer3000).

Example 2

20g of lurasidone was weighed, added into 400mL of absolute ethanol, stirred at 80°C until completely dissolved, the solution was cooled to room temperature (25°C) by controlling the cooling rate at 0.2°C/min, the precipitated crystals were filtered, subjected to suction filtration, followed by vacuum drying at 60°C for 12 hours (-0.07MPa to -0.08MPa), the yield was 93%.

The particle size detection results showed that the obtained lurasidone had a particle size of 71.769$\mu$m (Dv50, measured by Malvern Mastersizer 3000).

Example 3

20g of lurasidone was weighed, added into 300mL of isopropanol, stirred at 85°C until completely dissolved, the solution was cooled to 60°C by controlling the cooling rate at 0.05°C/min, then the solution was cooled to room temperature (25°C) by controlling the cooling rate at 0.2°C/min, the precipitated crystals were filtered, subjected to suction filtration, followed by vacuum drying at 60°C for 12 hours (-0.07MPa to -0.08MPa), and the yield was 97%.

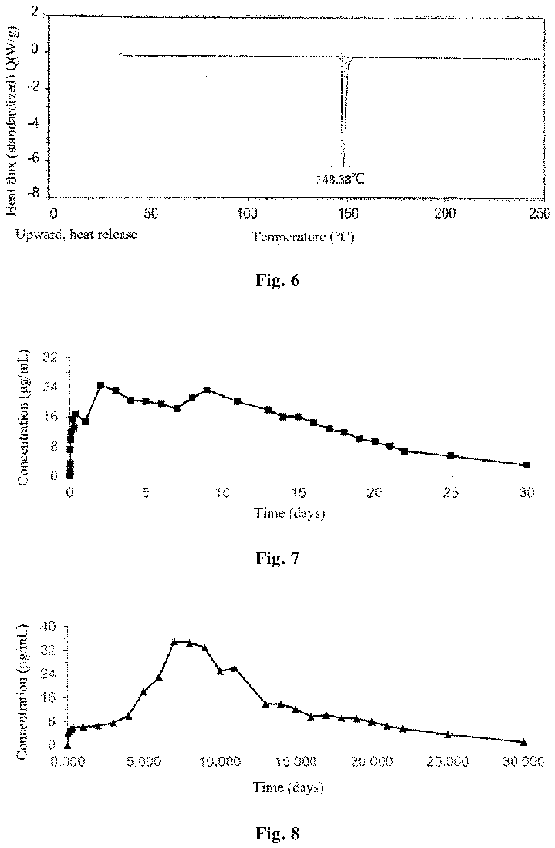

The XRD results (Figure 4) showed that the obtained lurasidone was consistent with the crystal form in Example 1; it was found that the lurasidone had irregular form observed with a polarizing microscope (CiPOL, Nikon) (Figure 5), it was consistent with Example 1; the DSC results showed that the melting point was 148.38°C (Figure 6); the particle size was 32.319$\mu$m (Dv50, measured by Malvern Mastersizer 3000).

Example 4

20g of lurasidone was weighed, added into 300mL of isopropanol, stirred at 85°C until completely dissolved, the solution was cooed to room temperature (25°C) by controlling the cooling rate at 0.2°C/min, the precipitated crystals were filtrated, subjected to suction filtration, followed by vacuum drying at 60°C for 12 hours (-0.07MPa to -0.08MPa), the yield was 97%.

The particle size detection results showed that the obtained lurasidone had a particle size of 108.333$\mu$m (Dv50, measured by Malvern Mastersizer 3000).

Based on the experimental results of Example 1 and Example 2, Example 3 and Example 4, it could be found that the particle size of the precipitated crystals could be controlled by controlling the cooling rate during the cooling process.

Example 5: Effects of shearing conditions on particle size

① 20g of lurasidone was weighed, added into 200mL of n-propanol, stirred at 80°C until completely dissolved, the temperature of the cooling medium was kept at 5°C until the solution was cooled to room temperature (25°C), the precipitated crystals were filtered, subjected to suction filtration, followed by vacuum drying at 60°C for 12 hours (-0.07MPa to -0.08MPa), the yield was 96%, and the product was marked as Sample 1.

② 20g of lurasidone was weighed, added into 200mL of n-propanol, stirred at 80°C until completely dissolved, the temperature of the cooling medium was kept at 5°C, and the shearer was turned on at the same time (line speed: 6.3m/s) until the solution was cooled to room temperature (25°C), the precipitated crystals were filtered, subjected to suction filtration, followed by vacuum drying at 60°C for 12 hours (-0.07MPa to -0.08MPa), the yield was 95%, and the product was marked as Sample 2.

③ 20g of lurasidone was weighed, added into 200mL of n-propanol, stirred at 80°C until completely dissolved, the temperature of the cooling medium was kept at 5°C, and the shearer was turned on at the same time (line speed: 12.6m/s) until the solution was cooled to room temperature (25°C), the precipitated crystals were filtered, subjected to suction filtration, followed by vacuum drying at 60°C for 12 hours (-0.07MPa to -0.08Mpa), the yield was 94%, and the product was marked as Sample 3.

The particle size detection results of Samples 1 to 3 were shown in Table 1, from which it could be concluded that under the same cooling conditions, the introduction of shear conditions could further control the particle size of the precipitated crystals, and the faster the shear line speed, the smaller the particle size of the precipitated crystals.

Table 1: Particle size distribution results of samples in Example 5

| Sample name | Particle size distribution | | |
|---|---|---|---|
| | Dv10 ($\mu$m) | Dv50 ($\mu$m) | Dv90 ($\mu$m) |
| Sample 1 | 12.100 | 42.270 | 120.524 |
| Sample 2 | 6.114 | 15.412 | 33.430 |
| Sample 3 | 4.927 | 11.436 | 24.500 |

Example 6: Effects of cooling medium temperature on particle size

① Preparation of Sample 4: same as Sample 2 in Example 5.

② Preparation of Sample 5: 20g of lurasidone was weighed, added into 200mL of n-propanol, stirred at 80°C until completely dissolved, the temperature of the cooling medium was kept at 0°C, and the shearer was turned on at the same time (line speed: 6.3m/s), until the solution was cooled to room temperature (25°C), the precipitated crystals were filtered, subjected to suction filtration, followed by vacuum drying at 60°C for 12h (-0.07MPa to -0.08MPa), the yield was 95%.

③ Preparation of Sample 6: 20g of lurasidone was weighed, added into 200mL of n-propanol, stirred at 80°C until completely dissolved, the temperature of the cooling medium was kept at - 10°C, and the shearer was turned on at the same time (line speed: 6.3m/s) until the solution was cooled to room temperature (25°C), the precipitated crystals were filtered, subjected to suction filtration, followed by vacuum drying at 60°C for 12 hours (-0.07MPa to -0.08MPa), and the yield was 96%.

The particle size detection results of Samples 4 to 6 were shown in Table 2, from which it could be concluded that under the same shear conditions, the lower the temperature of the cooling medium, the smaller the particle size of the precipitated crystals.

Table 2: Particle size distribution results of sample in Example 6

| Sample name | Particle size distribution | | |
|---|---|---|---|
| | Dv10 ($\mu$m) | Dv50 ($\mu$m) | Dv90 ($\mu$m) |
| Sample 4 | 6.114 | 15.412 | 33.430 |
| Sample 5 | 5.306 | 13.535 | 30.483 |
| Sample 6 | 4.551 | 10.963 | 25.217 |

Example 7: Effect of cooling medium temperature on crystallization temperature and particle size

① 20g of lurasidone was weighed, added into 200mL of n-propanol, stirred at 80°C until completely dissolved, the temperature of the cooling medium was kept at 60°C, the temperature at the time when the solution started to precipitate crystals was recorded, which was the crystallization temperature (64.2°C). The sample was stirred again at 80°C until completely dissolved, the temperature of the cooling medium was kept at 60°C (the first stage of cooling); the shearing (8000rpm) was started when the solution temperature dropped to 65.2°C; the temperature of the cooling medium was kept at 20°C (the second stage of cooling) when the solution temperature dropped to 60°C, the shearing was stopped when the solution temperature dropped to 25°C, the precipitated crystals were filtered, subjected to suction filtration, followed by vacuum drying at 40°C for 12 hours (-0.07MPa to -0.08MPa), and the product was marked as Sample 7.

② 20g of lurasidone was weighed, added into 200mL of n-propanol, stirred at 80°C until completely dissolved, the temperature of the cooling medium was kept at 55°C, the temperature at the time when the solution started to precipitate crystals was recorded, which was the crystallization temperature (61.4°C). The sample was stirred again at 80°C until completely dissolved, the temperature of the cooling medium was kept at 55°C (the first stage of cooling); the shearing (8000rpm) was started when the solution temperature dropped to 62.4°C; the temperature of the cooling medium was kept at 20°C (the second stage of cooling) when the solution temperature dropped to 55°C, the shearing was stopped when the solution temperature dropped to 25°C, the precipitated crystals were filtered, subjected to suction filtration, followed by vacuum drying at 40°C for 12 hours (-0.07MPa to -0.08MPa), and the product was marked as Sample 8. The experiment was repeated twice under this condition, and the obtained samples were marked as Sample 9 and Sample 10 in turn.

③ 20g of lurasidone free base was weighed, added into 200mL of n-propanol, stirred at 80°C until completely dissolved, the temperature of the cooling medium was kept at 50°C, the temperature at the time when the solution started to precipitate crystals was recorded, which was the crystallization temperature (59.1°C). The sample was stirred again at 80°C until completely dissolved, the temperature of the cooling medium was kept at 50°C (the first stage of cooling); the shearing (8000rpm) was started when the solution temperature dropped to 60.1°C; the temperature of the cooling medium was kept at 20°C (the second stage of cooling) when the solution temperature dropped to 55°C, the shearing was stopped when the solution temperature dropped to 25°C, the precipitated crystals were filtered, subjected to suction filtration, followed by vacuum drying at 40°C for 12 hours (-0.07MPa to -0.08Mpa), the product was marked as Sample 11.

The main experimental conditions and particle size detection results of Samples 7 to 11 were shown in Table 3, from which it could be concluded that the lower the temperature of the cooling medium (the first stage of cooling), the lower the crystallization temperature, and the smaller the particle size of the obtained samples (Dv50). At the same time, it could be seen that the preparation process had good reproducibility, and the particle sizes of the obtained samples were stable and controllable (Sample 8 to Sample 10).

Table 3: Main experimental conditions and particle size distribution results of samples in Example 7

| Sample name | Cooling medium temperature in the first stage of cooling (°C) | Crystallization temperature (°C) | Temperature for starting shearing (°C) | Sample particle size (Dv50) ($\mu$m) |
|---|---|---|---|---|
| Sample 7 | 60 | 64.2 | 65.2 | 17.967 |
| Sample 8 | 55 | 61.4 | 62.4 | 15.457 |
| Sample 9 | 55 | 61.4 | 62.4 | 15.562 |
| Sample 10 | 55 | 61.4 | 62.4 | 15.254 |
| Sample 11 | 50 | 59.1 | 60.1 | 10.599 |

Example 8

Taking Tween 20 as a representative, the wetting effects of HS 15 and commonly used surfactants on lurasidone were compared. The lurasidone prepared in Example 1 was added to HS 15 solution (1.2% (W/W)) and Tween 20 solution (1.2% (W/W)), respectively (which were marked as Test Sample 2, Test Sample 3, and Test Sample 1 was the lurasidone obtained in Example 1), stirred at 600 rpm for 50 minutes, then the particle size detection was performed (Malvern Mastersizer 3000), and the test results were shown in Table 4.

It could be seen from the results that under the same treatment conditions, lurasidone could be completely dispersed in the HS 15 solution, and the particle size of the sample was consistent with that of the raw material, but it could not be completely dispersed in the Tween 20 solution, there were still some particles agglomerated together so that the particle size of the sample was about 1.2 times the particle size of the raw material. It could be seen that the wetting effect of HS 15 on lurasidone was better than that of the commonly used surfactant Tween 20.

Table 4: Comparison of wetting effects of HS 15 and Tween 20 on lurasidone

| Test sample No. | Dispersing agent | Particle size distribution | | |
|---|---|---|---|---|
| | | Dv10 ($\mu$m) | Dv50 ($\mu$m) | Dv90 ($\mu$m) |
| 1 | / | 7.231 | 16.320 | 34.891 |
| 2 | 1.2% HS 15 | 5.189 | 16.195 | 35.181 |
| 3 | 1.2 % Tween-20 | 6.803 | 20.124 | 50.289 |

Example 9

Still taking Tween 20 as the representative, stabilizers commonly used by professionals in the art were selected to form complete formulations for injectable suspension, and the effects of HS 15 on the particle size stability of lurasidone were observed by comparison.

The lurasidone prepared in Example 1 was added to the prepared solution of excipients, dispersed uniformly to form a suspension solution, marked as Test Samples 4 to 9, which were immediately subjected to the particle size detection, and then the particle size detection was carried out again after being placed at room temperature for 20 days, and Dv50 was used as the reference standard, to compare the growth rate of particle size in 20 days. The formulations and investigation results the test samples were shown in Table 5.

According to the investigation results, for commonly used stabilizers, the stability of the formulation with HS 15 as the wetting agent was significantly better than that of the formulation with Tween 20 as the wetting agent, and HS 15 was more conducive to the stability of the particle size of the lurasidone suspension.

Table 5: Investigation results of particle size stability

| Test sample No. | | 4 | 5 | 6 | 7 | 8 | 9 |
|---|---|---|---|---|---|---|---|
| Formulation | API (W/W) | Lurasidone (30%) | | | | | |
| | Wetting agent (W/W) | Tween 20 (1.2%) | | | HS 15 (1.2%) | | |
| | Stabilizer (W/W) | PEG4000 (5%) | PVP K17 (0.5%) | PVP K30 (0.59%) | PEG4000 (5%) | PVP K17 (0.5%) | PVP K30 (0.59%) |
| | Buffer salt (W/W) | sodium dihydrogen phosphate (0.24%), disodium hydrogen phosphate (0.48%) | | | | | |
| | Dispersion medium | Water for injection | | | | | |
| Particle size growth rate after 20 days at room temperature | | 20.3% | 33.9% | 30.6% | 9.7% | 12.9% | 14.9% |
| Note:<br>1: PEG4000: polyethylene glycol 4000;<br>PVP K17: polyvinylpyrrolidone K17;<br>PVP K30: polyvinylpyrrolidone K30;<br>2: The formulation with PEG4000 as stabilizer also contained 0.83% (W/W) citric acid;<br>3: $$\text{Particle size growth rate (\%)} = \frac{Dv50 \text{ of Day } 20 - Dv50 \text{ of Day } 0}{Dv50 \text{ of Day } 0} \times 100\%$$ | | | | | | | |

Example 10

① Preparation of injectable lurasidone suspension: The lurasidone obtained in Example 1 was dispersed into an excipient solution (see Table 6 for the formulation, batch size: 30g) under shear conditions (3.4m/s, 10min). The particle size detection results showed that the particle size of the obtained sample was 16.612μm (Dv50), and it was marked as Test Sample 10.

Table 6: Formulation of Test Sample 10

| Name | Name Content (%, W/W) |
| --- | --- |
| Lurasidone | 30 |
| HS 15 | 1.2 |
| Sodium dihydrogen phosphate | 0.24 |
| Disodium hydrogen phosphate | 0.48 |
| Sodium carboxymethylcellulose | 1.25 |
| Water for injection | / |

② In vivo pharmacokinetic experiment in rats

Six SD rats (half male and half female, 180g to 220g) were intramuscularly injected with the lurasidone suspension (50mg/Kg) of Test Sample 10. Taking the day of administration as Day 0, 0.3ml of vein blood was collected (treated with EDTA-$K_2$ anticoagulant) respectively before administration, and 5min, 15min, 30min, 1h, 2h, 4h, 6h, 8h, 1 day (24h), 2 days (48h), 3 days (72h), 4 days (96h), 5 days (120h), 6 days ( 144h), 7 days (168h), 9 days (216h), 11 days (264h), 13 days (312h), 14 days (336h), 15 days (360h), 16 days (384h), 17 days (408h), 18 days (432h), 19 days (456h), 20 days (480h), 21 days (504h), 22 days (528h), 25 days (600h), 30 days (720h), 35 days (840h) after administration. The collected whole blood was temporarily stored in an ice box, and the plasma was separated by centrifugation at 4000 rpm for 10 minutes (4°C) within 2 hours, and the collected plasma was stored in a -80°C refrigerator for later test. The blood concentration of lurasidone was detected by LC-MS/MS method, and the results were shown in Figure 7.

Comparative Example 1:

Preparation of injectable lurasidone suspension:

a) Lurasidone hydrochloride was dissolved in 70% ethanol in water containing 1% (w/v) polysorbate 80 to reach a concentration of 2% (w/v) in a total volume of 500 ml (active ingredient solution);

b) An aqueous solution containing 0.7% (W/V) disodium hydrogen phosphate (anhydrous) and 1% (W/V) polysorbate 80 in a total mass of 500 g was separately prepared (crystallization agent);

c) The active ingredient solution was added dropwise to the crystallization agent at a flow rate of 50mL/min at 25°C, and the stirring speed was 675rpm;

d) The precipitated crystals were collected by filtration and the liquid components were removed;

e) preparation of a carrier solution, which contained 1.4% (W/W) HS 15, 0.28% (W/W) sodium dihydrogen phosphate, 0.57% (W/W) disodium hydrogen phosphate, and 1.5% (W/W) carboxymethyl sodium cellulose;

f) The collected crystals were suspended in the carrier solution, filtered and resuspended, this process was repeated for several times, the collected crystals were suspended in the carrier solution again, and finally a lurasidone suspension was prepared, which had the same formulation composition as Test Sample 10, and marked as Test Sample 11, and the detection showed that it had a particle size of 15.423μm (Dv50).

Six SD rats (half male and half female, 180g to 220g) were intramuscularly injected with the lurasidone suspension (50mg/Kg) of Test Sample 11. By taking the day of administration as Day 0, 0.3ml of vein blood was respectively collected (treated with EDTA-K2 anticoagulant) before administration, and 5min, 15min, 30min, 1h, 2h, 4h, 6h, 8h, 1 day (24h), 2 days (48h), 3 days (72h), 4 days (96h), 5 days (120h), 6 days ( 144h), 7 days (168h), 9 days (216h), 11 days (264h), 13 days (312h), 14 days (336h), 15 days (360h), 16 days (384h), 17 days (408h), 18 days (432h), 19 days (456h), 20 days (480h), 21 days (504h), 22 days (528h), 25 days (600h), 30 days (720h), 35 days (840h) after administration. The collected whole blood was temporarily stored in an ice box, and the plasma was separated by centrifugation at 4000 rpm for 10 minutes (4°C) within 2 hours, and the collected plasma was stored in a -80°C refrigerator for later test. The blood concentration of lurasidone was detected by LC-MS/MS method, and the results were shown in Figure 8.

According to the in vivo pharmacokinetic results of SD rats as shown in Figure 7 and Figure 8, both Test Sample 10 and Test Sample 11 could achieve the sustained-release effect for 30 days. However, after intramuscular injection of Test Sample 10, the plasma drug concentration increased rapidly (the plasma drug concentration was 62% of $C_{max}$ within about 4 hours), then the release became slow, so that an oral administration was not required at the beginning of treatment. However, after the intramuscular injection of Test Sample 11, the blood drug concentration increased slowly, and the tablets of the same drug should be taken orally after injection to quickly reach the therapeutic level of the blood drug concentration, therefore, the risk of missing doses in patients still existed. In addition, by controlling the particle size of lurasidone, its sustained release period could be regulated. Generally speaking, the larger the particle size, the longer the sustained-release time.

Example 12: Investigation of particle size stability of sample

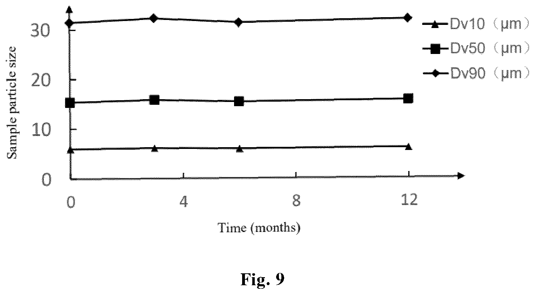

20g of lurasidone was weighed, added into 200mL of n-propanol, stirred at 80°C until completely dissolved, the temperature of the cooling medium was kept at 5°C, and the shearer was turned on (line speed: 6.3m/s) at the same time until the solution was cooled to room temperature (25°C), the precipitated crystals were filtered, washed with 1L of water for injection, and dispersed into a pre-prepared excipient solution, making its composition same as that of Test Sample 10 (Table 6), and it was marked as Test Sample 12. Its particle size was detected on day 0, 3 months, 6 months, 12 months thereafter (Figure 9). According to the detection results, the particle size did not change significantly in 12 months, indicating that the preparation had excellent stability.

Various modifications of the present invention, in addition to those described herein, will be apparent to those skilled in the art from the foregoing description. Such modifications are also intended to fall within the scope of the appended claims. Each reference cited in the present application, including all patents, patent applications, journal articles, books, and any other publications, is hereby incorporated by reference in its entirety.

## Claims

1. A lurasidone solid, which has a powder X-ray diffraction pattern showing the following diffraction angles $2\theta(°)$: $15.1\pm0.2$, $15.5\pm0.2$, $16.3\pm0.2$, $16.6\pm0.2$, $18.0\pm0.2$ and $20.0\pm0.2$, preferably, the solid has a powder X-ray diffraction pattern showing the following diffraction angles $2\theta(°)$: $11.2\pm0.2$, $15.1\pm0.2$, $15.5\pm0.2$, $16.3\pm0.2$, $16.6\pm0.2$, $18.0\pm0.2$, $19.1\pm0.2$, $19.5\pm0.2$, $20.0\pm0.2$, $20.9\pm0.2$, $22.3\pm0.2$ and $26.0\pm0.2$,

   and, the solid is in irregular form;
   preferably, the solid has a Dv50 particle size of 6μm to 110μm, such as 6μm to 100μm, 6μm to 90μm, 6μm to 80μm, 6μm to 70μm, 6μm to 60μm, 6μm to 50μm, 6μm to 40μm, 6μm to 30μm, 6μm to 20μm, 6μm to 10μm, 10μm to 100μm, 10μm to 90μm, 10μm to 80μm 10μm to 70μm, 10μm to 60μm, 10μm to 50μm, 10μm to 40μm, 10μm to 30μm, 10μm to 20μm, 20μm to 100μm, 20μm to 90μm, 20μm to 80μm 20μm to 70μm, 20μm to 60μm, 20μm to 50μm, 20μm to 40μm, 20μm to 30μm, 20μm to 100μm, 20μm to 90μm, 20μm to 80μm 20μm to 70μm, 20μm to 60μm, 20μm to 50μm, 20μm to 40μm, or 20μm to 30μm;
   preferably, the solid has a melting point of 148±5°C, such as 148±4°C, 148±3°C, 148±2°C, 148±1.5°C, 148±1°C, 148±0.9°C, 148±0.8°C, 148±0.7°C, 148±0.6°C, 148±0.5°C, 148±0.4°C, 148±0.3°C, 148±0.2°C or 148±0.1°C.

2. A method for preparing the solid according to claim 1, which comprises the following steps:

   (1) dissolving lurasidone in an organic solvent under heating to obtain a solution of lurasidone;
   (2) cooling the solution obtained in step (1) to room temperature (e.g., 25 °C) to precipitate crystals;
   (3) collecting the crystals obtained in step (2) to obtain the solid;

   optionally, during the cooling process as described in step (2), controlling stirring speed to 100 rpm to 2000 rpm, and/or shear line speed to 1 m/s to 32 m/s.

**3.** The method according to claim 2, which has one or more of the following features:

1) the heating refers to heating to a degree sufficient to increase the solubility of lurasidone in the organic solvent; preferably, heating to the boiling point of the organic solvent or to the temperature within ±1°C to ±30°C of its boiling point, for example within ±1°C, ±2°C, ±3°C, ±4°C, ±5°C, ±6°C, ±7°C, ±8°C, ±9°C, ±10°C, ±15°C, ±20°C, ±25°C or ±30°C of its boiling point;

2) the organic solvent is the Class III solvent classified according to ICH; preferably, the organic solvent is selected from the group consisting of ethanol, n-propanol, isopropanol, isopropyl acetate, methyl acetate, ethyl acetate, acetic acid, 1-pentanol, 1-butanol and 2-butanol; further preferably, the organic solvent is selected from the group consisting of ethanol, n-propanol, isopropanol and any combination thereof; further preferably, the organic solvent is ethanol or isopropanol;

3) after step (1) and before step (2), a step of filtering and sterilizing the solution, for example, a step of filtering and sterilizing by using a $0.22\mu$m filter membrane, is further comprised;

4) cooling the solution obtained in step (1) to room temperature at the following cooling rate: 0.05°C/min to 15°C/min, 0.05°C/min to 5°C/min, 0.05°C/min to 3°C/min, 0.05°C/min to 2°C/min, 0.05°C/min to 1°C/min, 0.05°C/min to 0.5°C/min, 0.05°C/min to 0.2°C/min, or 0.05°C/min to 0.1°C/min; or cooling the solution obtained in step (1) to room temperature in a cooling medium at -20 to 70°C; preferably, setting two cooling stages, wherein the temperature of the cooling medium in the first cooling stage is 50% to 90% of the heating temperature in step (1), and the temperature of the cooling medium in the second cooling stage is room temperature (e.g., 25°C);

5) during the cooling process as described in step (2), controlling the stirring speed to 100rpm to 1000rpm, such as 200rpm to 1000rpm, 300rpm to 1000rpm, 400rpm to 1000rpm, 500rpm to 1000rpm, 600rpm to 1000rpm, 700rpm to 1000rpm, 800rpm to 1000rpm, or 900rpm to 1000rpm;

6) during the cooling process as described in step (2), controlling the shear line speed to 1m/s to 16m/s, such as 1.5m/s to 16m/s, 2m/s to 16m/s, 2.5m/s to 16m/s, 3m/s to 16m/s, 3.5m/s to 16m/s, 4m/s to 16m/s, 4.5m/s to 16m/s, 5m/s to 16m/s, 5.5m/s to 16m/s, 6m/s to 16m/s, 6.5m/s to 16m/s, 7m/s to 16m/s, 7.5m/s to 16m/s, 8m/s to 16m/s, 8.5m/s to 16m/s, 9m/s to 16m/s, 9.5m/s to 16m/s, 10m/s to 16m/s, 10.5m/s to 16m/s, 11m/s to 16m/s, 11.5 m/s to 16m/s, 12m/s to 16m/s, 12.5m/s to 16m/s, 13m/s to 16m/s, 13.5m/s to 16m/s, 14m/s to 16m/s, 14.5m/s to 16m/s, 15m/s to 16m/s, or 15.5m/s to 16m/s;

7) during the cooling process as described in step (2), optionally, when the temperature of the solution obtained in step (1) drops to a specific temperature, the shearer is turned on for crystallization, and the specific temperature refers to a temperature that is 0 to 5°C higher than the crystallization temperature;

8) in step (3), after collecting the crystals obtained in step (2), a step of drying is optionally comprised, for example, heating drying or heating vacuum drying; preferably, vacuum drying at 40 to 70 °C for 6 to 24 hours; more preferably, vacuum drying at 40 to 60 °C for 6 to 12 hours;

9) in step (3), after collecting the crystals obtained in step (2), a step of washing is comprised, and the washing agent is water for injection; optionally, the washing agent contains HS 15, and its content is preferably 0.01% to 5% (W/W);

10) features of items 4) and 6);

11) features of items 4), 6) and 7);

12) features of items 1), 2), 4) and 6);

13) features of items 1), 2), 4), 6) and 7);

14) features of items 1), 2), 4), 6), 7) and 9);

15) features of items 1), 2), 3), 4), 6), 7) and 8);

16) features of items 1), 2), 3), 4), 6), 7) and 9).

**4.** A pharmaceutical composition, which comprises the lurasidone solid according to claim 1, and a pharmaceutical excipient, such as one or more of a surfactant, a pH buffering agent, a dispersing agent, a pH regulator, an osmotic pressure regulator and a stabilizer.

**5.** The pharmaceutical composition according to claim 4, which comprises 10% to 50% (W/W) of the lurasidone solid.

**6.** The pharmaceutical composition according to claim 4 or 5, wherein the surfactant is a nonionic surfactant, such as one or more of polysorbate 80, polysorbate 20, polyoxyethylene castor oil, sorbitan fatty acid ester, succinate and Kolliphor® HS 15, preferably Kolliphor® HS 15; preferably, the content of the surfactant accounts for 0.01% to 5% (W/W) of the pharmaceutical composition;

preferably, the pH buffering agent is one or more selected from the group consisting of sodium phosphate, disodium hydrogen phosphate, sodium dihydrogen phosphate, potassium phosphate, dipotassium hydrogen phosphate, potassium dihydrogen phosphate, tromethamine, sodium carbonate, sodium acetate, sodium bicarbonate, meglumine, arginine, triethanolamine, citric acid and acetic acid; preferably, a combination of sodium dihydrogen phosphate and disodium hydrogen phosphate; preferably, the content of the pH buffing agent accounts for 0.01% to 5% (W/W) of the pharmaceutical composition;

preferably, the dispersing agent is an aqueous medium, preferably water for injection;

preferably, the pH regulator is selected from the group consisting of sodium hydroxide, potassium hydroxide, hydrochloric acid, and aqueous solution thereof; preferably, the pH regulator has a content that makes pH of the pharmaceutical composition 4 to 9;

preferably, the osmotic pressure regulator is selected from the group consisting of sodium chloride, glucose and mannitol; preferably, the content of the osmotic pressure regulator accounts for 0.1% to 10% (W/W) of the pharmaceutical composition;

preferably, the stabilizer is selected from the group consisting of polyethylene glycol stabilizer (e.g., polyethylene glycol 4000), carboxymethylcellulose and sodium salt thereof, polyvinylpyrrolidone (e.g., PVP K17, PVP K30), hypromellose, methylcellulose, poloxamer (e.g., 188, 407) and polyvinyl alcohol; preferably, the content of the stabilizer accounts for 0.01% to 25% (W/W) of the pharmaceutical composition.

7. The pharmaceutical composition according to any one of claims 4 to 6, which can pass through a syringe needle with an inner diameter of 0.3mm to 1mm (e.g., about 0.3mm, about 0.4mm, about 0.5mm, about 0.6mm, about 0.7mm, about 0.8mm, about 0.9mm, or about 1mm);

preferably, the pharmaceutical composition is an injection, preferably a long-acting sustained-release injection, such as a suspension injection or a lyophilized preparation;

preferably, the pharmaceutical composition exists in the form of a unit preparation, and further preferably, the unit preparation contains 50 mg to 500 mg of the lurasidone solid.

8. The pharmaceutical composition according to any one of claims 4 to 7, which has a weight composition of:

| | |
|---|---|
| Lurasidone solid | 30%; |
| Kolliphor® HS 15 | 1.2%; |
| Sodium dihydrogen phosphate | 0.24%; |
| Disodium hydrogen phosphate | 0.48%; |
| Sodium carboxymethyl cellulose | 1.25%; and |
| Water for injection | balanced. |

9. A method for preparing the pharmaceutical composition according to any one of claims 4 to 8, which comprises the following steps:

dispersing the lurasidone solid into the pharmaceutical excipient to obtain the pharmaceutical composition;
preferably, during the dispersing process, an operation of stirring, shearing, sonication, or any combination thereof is conducted;
preferably, the speed of the stirring is 100 to 2000rpm;
preferably, the line speed of the shearing is 1m/s to 32m/s;
preferably, the frequency of the sonication is 20 to 60 KHz.

10. Use of the lurasidone solid according to claim 1 or the pharmaceutical composition according to any one of claims 4 to 8 in the manufacture of a medicament for the treatment of a mental disease; preferably, the mental disease is selected from the group consisting of schizophrenia syndrome, bipolar disorder, and depression.

11. The lurasidone solid according to claim 1 or the pharmaceutical composition according to any one of claims 4 to 8, for use in the treatment of a mental disease; preferably, the mental disease is selected from the group consisting of schizophrenia, bipolar disorder and depression.

12. A method of treating a mental disease, which comprises administering to a subject in need thereof a therapeutically effective amount of the lurasidone solid according to claim 1 or the pharmaceutical composition according to any one of claims 4 to 8; preferably, the mental disease is selected from the group consisting of schizophrenia, bipolar disorder and depression.

**Fig. 1**

**Fig. 2**

**Fig. 3**

**Fig. 4**

**Fig. 5**

**Fig. 6**

**Fig. 7**

**Fig. 8**

**Fig. 9**

**EP 4 317 156 A1**

## INTERNATIONAL SEARCH REPORT

| | International application No. |
|---|---|
| | **PCT/CN2022/081937** |

| A. | CLASSIFICATION OF SUBJECT MATTER |
|---|---|

C07D 417/12(2006.01)i；　A61K 9/10(2006.01)i；　A61K 47/10(2006.01)i；　A61K 31/496(2006.01)i；　A61P 25/00(2006.01)i；　A61P 25/18(2006.01)i；　A61P 25/24(2006.01)i

According to International Patent Classification (IPC) or to both national classification and IPC

| B. | FIELDS SEARCHED |
|---|---|

Minimum documentation searched (classification system followed by classification symbols)

　　C07D; A61K; A61P

Documentation searched other than minimum documentation to the extent that such documents are included in the fields searched

Electronic data base consulted during the international search (name of data base and, where practicable, search terms used)

　　CNTXT, VEN, ENTXT, DWPI, CNKI, 万方, WANFANG, 百度, BAIDU, caplus, marpat, registry: 科伦, 李明, 梁相永, 苏正兴, 李丹, 柯朵, 易聪, 魏巍, 邓贵福, 彭娅, 赵栋, 王晶翼, 鲁拉西酮, 罗舒达, 晶体, 固体, 混悬, 注射, lurasidone, Latuda, cryst?, solid, suspen?, inject?, 367514-87-2.

| C. | DOCUMENTS CONSIDERED TO BE RELEVANT |
|---|---|

| Category* | Citation of document, with indication, where appropriate, of the relevant passages | Relevant to claim No. |
|---|---|---|
| X | US 2015018368 A1 (CADILA HEALTHCARE LTD.) 15 January 2015 (2015-01-15) entire document, in particular embodiment 11, description, paragraphs [0003] and [0075]-[0078], and figures 3 and 4 | 1-11 |
| X | CN 107759583 A (TIANJIN HANKANG PHARMACEUTICAL BIOTECHNOLOGY CO., LTD.) 06 March 2018 (2018-03-06) entire document, in particular embodiment 2 | 1-11 |
| X | US 2019336439 A1 (LIFEMAX LABORATORIES, INC.) 07 November 2019 (2019-11-07) entire document, in particular description, paragraphs [0006]-[0016] and [0048], and embodiments 1-10 | 1-11 |
| X | CN 103249416 A (SUMITOMO DAINIPPON PHARMA CO., LTD.) 14 August 2013 (2013-08-14) entire document, in particular description, paragraphs [0111]-[0271] | 1-11 |
| X | CN 106916151 A (SUZHOU ERYE PHARMACEUTICAL CO., LTD.) 04 July 2017 (2017-07-04) entire document, in particular embodiments 1-3 | 1-11 |

☑ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **20 May 2022** | **01 June 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

23

**EP 4 317 156 A1**

**INTERNATIONAL SEARCH REPORT**

International application No.

**PCT/CN2022/081937**

## C. DOCUMENTS CONSIDERED TO BE RELEVANT

| Category* | Citation of document, with indication, where appropriate, of the relevant passages | Relevant to claim No. |
|---|---|---|
| X | WO 2014102834 A2 (HETERO RESEARCH FOUNDATION et al.) 03 July 2014 (2014-07-03)<br>  entire document, in particular embodiments 2-4 | 1-11 |
| X | US 2017246165 A1 (JUBILANT GENERICS LTD FORMERLY JUBILANT LIFE SCIENCES DIVISION) 31 August 2017 (2017-08-31)<br>  entire document, in particular embodiment 7 | 1-11 |
| X | WO 2014064714 A2 (GLENMARK PHARMACEUTICALS LTD. et al.) 01 May 2014 (2014-05-01)<br>  entire document, in particular embodiment 3 | 1-11 |
| X | US 2014243529 A1 (EDMOND PHARMA SRL) 28 August 2014 (2014-08-28)<br>  entire document, in particular embodiments 1-2 | 1-11 |
| X | CN 110734434 A (HUNAN DONGTING PHARMACEUTICAL CO., LTD.) 31 January 2020 (2020-01-31)<br>  entire document, in particular embodiments 1-5 | 1-11 |
| X | US 2015361099 A1 (JOHNSON MATTHEY PLC.) 17 December 2015 (2015-12-17)<br>  entire document, in particular embodiment 3 | 1-11 |
| X | US 2017349601 A1 (PIRAMAL ENTERPRISES LIMITED) 07 December 2017 (2017-12-07)<br>  entire document, in particular embodiment 2 | 1-11 |
| X | US 2014256939 A1 (DAINIPPON SUMITOMO PHARMA CO.) 11 September 2014 (2014-09-11)<br>  entire document, in particular embodiment 6 | 1-11 |
| X | WO 2017154021 A1 (ZCL CHEMICALS LTD.) 14 September 2017 (2017-09-14)<br>  entire document, in particular embodiments 1-3 | 1-11 |
| X | CN 107936007 A (CHANGZHOU YINSHENG PHARMACEUTICAL CO., LTD.) 20 April 2018 (2018-04-20)<br>  entire document, in particular embodiments 1-3 | 1-11 |
| X | CN 106146486 A (SHANGHAI PHARMACEUTICALS HOLDING CO., LTD.) 23 November 2016 (2016-11-23)<br>  entire document, in particular embodiments 1-3 | 1-11 |

24

**INTERNATIONAL SEARCH REPORT**

| International application No. |
|---|
| **PCT/CN2022/081937** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **12**
   because they relate to subject matter not required to be searched by this Authority, namely:

   [1]   Claim 12 relates to a method for treatment of a human or animal body, which belongs to the cases as
        defined in PCT Rule 39.1(iv).

2. ☐ Claims Nos.:
   because they relate to parts of the international application that do not comply with the prescribed requirements to such an
   extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2022/081937**

| Patent document cited in search report | | | Publication date (day/month/year) | Patent family member(s) | | | Publication date (day/month/year) |
|---|---|---|---|---|---|---|---|
| US | 2015018368 | A1 | 15 January 2015 | WO | 2013121440 | A1 | 22 August 2013 |
| | | | | US | 9409899 | B2 | 09 August 2016 |
| CN | 107759583 | A | 06 March 2018 | None | | | |
| US | 2019336439 | A1 | 07 November 2019 | WO | 2019213286 | A1 | 07 November 2019 |
| | | | | US | 10987303 | B2 | 27 April 2021 |
| CN | 103249416 | A | 14 August 2013 | CN | 107625728 | A | 26 January 2018 |
| | | | | JP | 2013543481 | A | 05 December 2013 |
| | | | | JP | 2016128503 | A | 14 July 2016 |
| | | | | CN | 107595771 | A | 19 January 2018 |
| | | | | US | 2016158228 | A1 | 09 June 2016 |
| | | | | WO | 2012053654 | A1 | 26 April 2012 |
| | | | | US | 2012091022 | A1 | 19 April 2012 |
| | | | | EP | 2629775 | A1 | 28 August 2013 |
| | | | | CA | 2814840 | A1 | 26 April 2012 |
| | | | | JP | 2019006822 | A | 17 January 2019 |
| | | | | KR | 20130139978 | A | 23 December 2013 |
| | | | | JP | 2017206543 | A | 24 November 2017 |
| | | | | US | 2019010149 | A1 | 10 January 2019 |
| | | | | CN | 103249416 | B | 04 June 2019 |
| | | | | EP | 2629775 | A4 | 11 June 2014 |
| | | | | JP | 5893616 | B2 | 23 March 2016 |
| | | | | US | 9469630 | B2 | 18 October 2016 |
| | | | | JP | 6173502 | B2 | 02 August 2017 |
| | | | | JP | 6410889 | B2 | 24 October 2018 |
| | | | | KR | 101936968 | B1 | 09 January 2019 |
| CN | 106916151 | A | 04 July 2017 | None | | | |
| WO | 2014102834 | A2 | 03 July 2014 | None | | | |
| US | 2017246165 | A1 | 31 August 2017 | EP | 3207041 | A1 | 23 August 2017 |
| | | | | WO | 2016059649 | A1 | 21 April 2016 |
| WO | 2014064714 | A2 | 01 May 2014 | None | | | |
| US | 2014243529 | A1 | 28 August 2014 | US | 8853395 | B2 | 17 October 2014 |
| CN | 110734434 | A | 31 January 2020 | None | | | |
| US | 2015361099 | A1 | 17 December 2015 | CA | 2951917 | A1 | 23 December 2015 |
| | | | | EP | 3154967 | A1 | 19 April 2017 |
| | | | | US | 2018099982 | A1 | 12 April 2018 |
| | | | | JP | 2017522284 | A | 10 August 2017 |
| | | | | US | 2018312530 | A1 | 01 November 2018 |
| | | | | WO | 2015195478 | A1 | 23 December 2015 |
| | | | | US | 9790237 | B2 | 17 October 2017 |
| | | | | US | 9957283 | B1 | 01 May 2018 |
| | | | | EP | 3154967 | B1 | 29 July 2020 |
| | | | | JP | 6594351 | B2 | 23 October 2019 |
| US | 2017349601 | A1 | 07 December 2017 | EP | 3242876 | A1 | 15 November 2017 |
| | | | | WO | 2016110798 | A1 | 14 July 2016 |
| | | | | US | 10196400 | B2 | 05 February 2019 |
| | | | | EP | 3242876 | A4 | 02 January 2019 |
| US | 2014256939 | A1 | 11 September 2014 | None | | | |
| WO | 2017154021 | A1 | 14 September 2017 | IN | 201621008234 | A | 01 April 2016 |

Form PCT/ISA/210 (patent family annex) (January 2015)

**INTERNATIONAL SEARCH REPORT**
Information on patent family members

International application No.

**PCT/CN2022/081937**

| Patent document cited in search report | | | Publication date (day/month/year) | Patent family member(s) | | | Publication date (day/month/year) |
|---|---|---|---|---|---|---|---|
| CN | 107936007 | A | 20 April 2018 | CN | 107936007 | B | 25 May 2021 |
| CN | 106146486 | A | 23 November 2016 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110348916
- CN 109998991 A
- WO 2019213286 A1
- CN 103249416 B
- CN 103249416 A